# EUROPEAN PATENT SPECIFICATION

(11) **EP 4 266 882 B1**
(45) Date of publication and mention of the grant of the patent: **08.10.2025**
(21) Application number: 21831031.6
(22) Date of filing: 13.12.2021
(51) Int. Cl.: A01N 37/02, A01N 59/16, A01P 1/00, A61K 8/36, A61K 31/19

(54) **ANTIMICROBIAL USAGE**
ANTIMIKROBIELLE VERWENDUNG
UTILISATION ANTIMICROBIENNE

(30) Priority: 23.12.2020 EP 20020650
(43) Date of publication of application: 01.11.2023
(73) Proprietor: Unilever IP Holdings B.V., 6708 WH Wageningen (NL); Unilever Global IP Limited, Wirral, Merseyside CH62 4ZD (GB)
(72) Inventor: JAMES, Alexander, Gordon, Bedford Bedfordshire MK44 1LQ (GB); MCWALTER, Joy, Rachel, Leeds Yorkshire LS14 2AR (GB); WEDDELL, Iain, Andrew, Wirral Merseyside CH63 3JW (GB); CORNMELL, Robert, Joseph, Wirral Merseyside CH63 3JW (GB)
(74) Representative: Whaley, Christopher
(86) International application number: PCT/EP2021/085536
(87) International publication number: WO 2022/136009

(56) References cited:
- WO-A1-2014/083330
- WO-A1-2018/087147
- PASQUET JULIA ET AL: "The contribution of zinc ions to the antimicrobial activity of zinc oxide", COLLOIDS AND SURFACES A : PHYSIOCHEMICAL AND ENGINEERINGS ASPECTS, ELSEVIER, AMSTERDAM, NL, vol. 457, 2 June 2014 (2014-06-02), pages 263 - 274, XP029037289, ISSN: 0927-7757, DOI: 10.1016/J.COLSURFA.2014.05.057
- ÅGREN M ET AL: "Zinc Oxide Inhibits Axillary Colonization by Members of the Genus Corynebacterium and Attenuates Self-perceived Malodour: A Randomized, Double-blind, Placebo-controlled Trial", ACTA DERMATO VENEREOLOGICA, vol. 100, no. 10, 4 May 2020 (2020-05-04), pages adv00145, XP055896173, Retrieved from the Internet <URL:https://www.medicaljournals.se/acta/content_files/files/pdf/100/10/5754.pdf> DOI: 10.2340/00015555-3499

## Description

### Field of Invention

The present invention is in the field of antimicrobial formulations, in particular antimicrobial deodorant compositions for topical application to the human body.

### Background

Antimicrobial compositions have been used for many years in multiple fields including medicine, food preservation and cosmetic preparations. Antimicrobial deodorant compositions have been used to reduce body malodour by topical application to the skin of the human body.

A wide range of antimicrobial agents have been discovered and used over the decades, including selected zinc salts.

Zinc oxide, particularly nanoparticulate zine oxide, has been used in antibacterial treatments [Y. Xie, et al, Applied Environmental Microbiology, 77 (2011), 2325], including use in food preservation [R. G. S. Chandra et al, Int. J. Sci. Research, 01(44) (2012), 524]. The latter reference also discloses the antimicrobial activity of zinc acetate and zinc hydroxyacetate. Zinc oxide has been reported to inhibit axillary colonisation by *Corynebacterium* and *Staphylococcus hominis,* with a concurrent reduction in malodour [Ågren et al, Acta Dermato-Venereologica, 100(10) (2020), adv00145].

US 4,565,693 (Marshner, 1986) discloses the use of zinc glycinate as a deodorant active material having the dual function of chemically neutralizing body odours and inhibiting bacterial growth, particularly of gram negative bacteria.

US 3,325,367 (Gillette Co., 1967) discloses zinc sulfamate and zinc phenol sulfamate as antimicrobial stringent metal salts useful in antiperspirant compositions.

Other publications disclose zinc salts of long chain fatty acids as deodorant actives; however these publications disclose the mechanism of action as being odour capture or adsorption, rather than antimicrobial affected deodorancy.

H. Kuhn et al, J. Surfact. Deterg. 3 (2000), 335-343, discloses the deodorancy mechanism of action zinc ricinoleate as being odour absorption.

WO 18/087147 and WO 18/087148 (Givaudan, 2018) disclose the use of zinc neodecanoate as a deodorant active, the mechanism of action being compared with that of zinc ricinoleate (*vide supra*).

### Summary of Invention

It is an object of the present invention to provide a highly effective use of a zinc salt as an antimicrobial agent.

It is a further object of the present invention to provide a mechanism of manipulating the microbiome on the skin to deliver a deodorancy benefit, i.e. a reduction in body malodour.

In a first aspect of the present invention, there is provided the non-therapeutic use of a zinc carboxylate salt for reducing microbial numbers on the surface of the mammalian body, particularly the human body, characterised in that the zinc carboxylate is a salt of a fatty acid having from 6 to 14 carbon atoms.

In a second aspect of the present invention, there is provided a zinc carboxylate salt for therapeutic use as an antimicrobial on the surface of human body, characterised in that the zinc carboxylate is a salt of a fatty acid having from 6 to 14 carbon atoms.

In a third aspect of the present invention, there is provided the non-therapeutic use of a zinc carboxylate salt to provide double protection from malodour via reducing microbial numbers on the surface of human body and entrapping odour molecules, characterised in that the zinc carboxylate is a salt of a fatty acid having from 6 to 14 carbon atoms.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, "ambient conditions" refers to about 20°C and 1 atmosphere pressure, unless otherwise indicated.

Herein, all numbers, amounts and ratios may optionally be understood to be modified by the word "about", unless otherwise indicated.

Herein, all percentages, ratios and amounts are by weight, unless otherwise indicated.

Herein, the word "comprising" is intended to mean "including" but not necessarily "consisting of", i.e., it is non-exhaustive.

In the first aspect of the invention referred to above, the non-therapeutic use may otherwise by described as a "cosmetic use", is typically achieved by the topical application of a cosmetic composition comprising a zinc carboxylate salt as specified. More is said concerning the cosmetic compositions that may be employed in the paragraphs that follow.

The topical application of a cosmetic composition comprising a zinc carboxylate salt as specified and as referred to in the above paragraph is preferably to the underarm regions of the human body. These are the regions most commonly in need of malodour reduction and also regions where *Staphylococcus hominis* bacteria are particularly prevalent.

The reduction of microbial numbers attained by the present invention is typically used to achieve a deodorancy benefit, i.e. a reduction in malodour on the skin of the mammalian body and particularly on the human body.

The microbial numbers reduced by the present invention are typically bacterial numbers, preferably *Staphylococcus* bacterial numbers and more preferably *Staphylococcus hominis* bacterial numbers. By reducing microbial numbers, the microbial transformation of non-odorous precursors into malodourous molecules can be reduced, as described by Lederberg and McCray in Scientist, 15 (2001), 8-9, for example.

By reducing the numbers of *Staphylococcus hominis* bacteria on the surface of the human body, a beneficial modification in the skin microbiome may be achieved, with regard to malodour reduction. *Staphylococcus hominis* bacteria have been identified as key bacteria in the formation of malodour on the skin of the human body [see Bawdon et al, FEMS Microbiol. Lett. 362, fnv111 (2015)]. Hence, reducing the number of these bacteria is important in reducing body malodour.

In preferred embodiments of the invention, the zinc carboxylate salt also entraps or captures malodour molecules, enabling double protection from malodour. This "entrapment" or "capture" may be by any mechanism, including absorption, adsorption, and by chemical reaction with the malodour molecules.

The zinc carboxylate salt used in the present invention is a salt of a fatty acid having from 6 to 14 carbon atoms. Such salts are relative hydrophobic in nature, enhancing the ease with which they can be formulated with other hydrophobic components typically used in cosmetic compositions (*vide infra*).

Herein, references to the "fatty acid having from 6 to 14 carbon atoms", etc., mean that the zinc carboxylate salt comprises at least one C6-C14 fatty acid carboxylate moiety.

In preferred embodiments, the zinc carboxylate salt has two C6-C14 fatty acid carboxylate moieties.

The preferred features detailed for the C6-C14 fatty acid in the following paragraph apply especially when the zinc carboxylate salt is a salt of two such C6-C14 fatty acids.

The zinc carboxylate salt used in the present invention are not as hydrophobic and of such high melting as zinc ricinoleate, which is particularly difficult to formulate because of its hydrophobicity and high melting point.

Preferred zinc carboxylates used in the present invention are liquids at 20°C and atmospheric pressure, i.e. they have a melting point of less than 20°C. This aids their formulation into cosmetic compositions.

Preferred zinc carboxylate salt used in the present invention are salts of a saturated fatty acid.

Preferred zinc carboxylate salt used in the present invention are salts of a fatty acid having from 8 to 12 carbon atoms, particularly when saturated.

Preferred zinc carboxylate salts used in the present invention are salts of a branched chain fatty acid, particularly when having from 8 to 12 carbon atoms and more particularly when saturated.

An especially preferred zinc carboxylate used in the present invention is zinc neodecanoate, which is a salt of zinc and neodecanoic acid having the formula [C₉H₂₀-CO.O]₂Zn.

In the second aspect of the invention, the zinc carboxylate salt is for therapeutic use as an antimicrobial on the surface of human body. In such use, all of the preferences described above with regard to the first aspect of the invention apply equally.

Uses according to the present invention are typically achieved through topical application of a cosmetic composition comprising a zinc carboxylate salt as specified and as mentioned above. Various preferred features of such compositions are detailed in the following paragraphs.

The compositions used to apply the zinc carboxylate may be of any format and may comprise any of the components typically used in such compositions. A preferred format for use is an aerosol spray format.

Preferred compositions used to apply the zinc carboxylate contain less than 2% by weight of water, more preferably they contain less than 0.5% of water, and most preferably they are water-free, i.e. completely anhydrous. This aids compatibility with and delivery of the zinc carboxylate.

In compositions used to apply the zinc neodecanoate, the zinc neodecanoate is preferably present at from 0.1 to 20%, more preferably at from 5 to 15% and most preferably at from 7 to 15%, ignoring any volatile propellant that may be present in the composition.

In preferred embodiments, the composition used to apply the zinc carboxylate comprises a fragrance. This may give rise to a deodorant composition having a triple mode of action: reducing microbial numbers, entrapment of malodours and malodour masking.

Encapsulated fragrances may also be advantageously employed, particularly those that are friable, i.e. released by the application of shear forces, such as those that may be encountered in the underarm regions of the human body.

In compositions comprising a fragrance, the total amount of fragrance is preferably up to 5% and more preferably up to 3% and the total amount of fragrance is preferably from 0.1%, more preferably from 0.5%, ignoring any volatile propellant present in the composition. In a particularly preferred embodiment, the total amount of fragrance is from 0.5 to 3%, again ignoring any volatile propellant that may be present in the composition.

The composition used to apply the zinc carboxylate is preferably a homogeneous, solution, this aid the effective delivery of the zinc carboxylate.

A carrier fluid is common component of compositions used in the present invention. Herein, "carrier fluid" refers to components of compositions used in the invention other than the zinc carboxylate and any volatile propellant that that may be present. Such carrier fluids are capable of flow at 20°C and atmospheric pressure. The amount of carrier fluid used in compositions used in the present invention is preferably from 25 to 95%, more preferably from 50 to 95% and most preferably from 65 to 90% of the composition, ignoring any volatile propellant that may be present in the composition.

An oil is a preferred component of carrier fluids used in compositions aid the present invention. Herein, "oils" are water-immiscible liquids having a melting point above 20°C.

Preferred oils are hydrocarbon oils, in particular saturated hydrocarbon oils having from 10 to 14 carbon atoms.

Other preferred oils are ester or ether oils. Examples of suitable ester oils include oils containing one or two alkyl groups of 12 to 24 carbon atoms length, including isopropyl myristate, isopropyl palmitate and myristyl palmitate. Other examples are liquid triglyceride oils, such as sunflower seed oil. Yet other suitable ester oils are alkyl or aryl benzoates such C₁₂₋₁₅ alkyl benzoate.

Examples of suitable ether oils include polypropylene glycol alkyl ethers such as PPG-14-butylether and PPG-15-stearyl ether.

When an aerosol spray compositions is used in the delivery of the present invention, a volatile propellant is typically employed. Herein, a volatile propellant is one having a boiling point of less than 10°C and one that may preferably be liquified at pressures within a typical aerosol spray can, i.e. at from 1.5 to 3 bar.

Typical volatile propellants are hydrocarbon or halogenated hydrocarbon gas (particularly fluorinated hydrocarbons such as 1,1-difluoroethane and/or 1-trifluoro-2-fluoroethane). It is preferred that the volatile propellant comprises liquefied hydrocarbon gases, especially C₃ to C₆ hydrocarbons, including propane, isopropane, butane, isobutane, and mixtures of two or more thereof.

A preferred component of some compositions used in the present invention is an antioxidant, such as BHT (butylated hydroxytoluene) or dilauryl thiodipropionate, typically used at a level of from 0.01 to 5%, excluding any volatile propellant that may be present.

### Examples

The materials tested in this study were:
Zinc neodecanoate [ZincZap^{™} (INCI: Zinc neodecanoate, Isopropyl myristate, Parfum)] and Ethylhexylglycerol [Sensiva SC 50^{®} (INCI: Ethylhexylglycerin)].

The antimicrobial activity of zinc neodecanoate was determined using an *in-vitro* model for bacterial growth on human stratum corneum, based on that described by van der Krieken et al. in Acta Derm. Venerol., 96 (2016), 873-879. The assay used human foot callus as a physical substrate and nutrient source for the growth of the test bacterium, *S. hominis* J23 [Bawdon et al, FEMS Microbiol. Lett. 362, fnv111 (2015)]. Callus was collected from healthy volunteers using an electronic foot file. Collected material was pooled into a single combined sample, frozen in liquid nitrogen, and ground into a fine powder using a mortar and pestle. This was accurately weighed into ca. 20 mg aliquots which were sterilised by gamma-irradiation prior to storage at -20°C until required. Prior to use, each aliquot was suspended in phosphate-buffered saline to a final concentration of 20 mg ml⁻¹.

For the assay set-up, 1 ml of 10 mg ml⁻¹ agarose was initially dispensed into each well of a 24-well microtitre plate and left to dry overnight in a microbiological safety cabinet. Wells were then loaded with 75 µl callus suspension (thoroughly agitated beforehand, and spread uniformly) and left to dry for a further 3 h. The bacterial inoculum was prepared from a fresh overnight culture of *S. hominis* grown on Tryptone Soya Agar (Thermo Fisher Scientific UK Ltd, Altrincham, UK), resuspended in normal saline to an optical density (OD620) of ca. 0.8, equivalent to ca. 108 colony-forming units (CFU) ml⁻¹. 40 µl of this bacterial suspension was then added to each well of the microtitre plate (apart from some cell-free controls). The test formulations (30 µl per well) were next added to the plate (apart from some cells-only and cell-free controls), either ethylhexylglycerol or zinc neodecanoate in ethanol, with each treatment (and control) run in quadruplicate. Finally, 25 µl resazurin solution (1 mg ml⁻¹) was added to each well, and the microtitre plate agitated using a magnetic shaker, prior to incubation at 37°C for 48 h. Fluorescence measurements (excitation at 550 nm; emission at 600 nm) were recorded at 24 h and 48 h using a Varioskan^{™} microplate reader (Thermo Fisher Scientific UK Ltd), and the mean value calculated from the four replicates for each treatment and control.

The assay works on the principle that the irreversible reduction of weakly fluorescent resazurin to the highly fluorescent resorufin is proportional to the level of aerobic respiration, and thus growth by metabolically active bacteria.

The methodology described above was used to determine the antimicrobial activity of ethanolic solutions of zinc neodecanoate and ethylhexylglycerol. The results are illustrated in Figure 1 and show that the zinc neodecanoate solution completely inhibited growth of *S. hominis* J23 on stratum corneum, providing an equivalent fluorescence value to the cells-free control after both 24 h and 48 h incubation. By contrast, the ethylhexylglycerol solution had no effect on the growth of *S. hominis* J23 on stratum corneum, providing an equivalent fluorescence value to the cells-only control after both 24 h and 48 h incubation.

In a further study, qPCR data was generated using species-level assays for *S. epidermidis,* the dominant bacterium on axillary skin but a non-producer of thioalcohols, and *S. hominis,* the second most abundant staphylococcal species in the underarm and the primary causal organism of thioalcohol-based malodour [Bawdon et al, FEMS Microbiol. Lett. 362, fnv111 (2015)]. Microbial sampling was carried 24 h and 48 h after topical application of zinc neodecanoate from an aerosol formulation¹, as described by Taylor et al in Int. J. Cosmet. Sci., 25 (2003), 137-145.
1. The aerosol composition comprised propellant (butane, isobutane, propane), cyclopentasiloxane, PPG-butyl ether, zinc neodecanoate, isopropyl myristate, parfum, C₁₂₋₁₅ alkyl benzoate, sunflower seed oil, octyldodecanol and BHT.

The results from the qPCR study are illustrated in Figure 2 and show that the zinc neodecanoate aerosol composition caused significant (ca. 85%) reductions in both S. *epidermidis* and *S. hominis* at 24 h; however, at the 48 h timepoint, the decline in *S. epidermidis* (42.4%) was lowered to a directional trend, while the reduction in *S. hominis* was maintained at a statistically significant level of 87.3%. This result indicates that zinc neodecanoate shows some selectivity in its *in-vivo* antimicrobial activity, targeting the thioalcohol-producing bacterium *S. hominis* to a greater extent than the dominant non-thioalcohol producing bacterium *S. epidermidis.*

## Claims

1. Non-therapeutic use of a zinc carboxylate salt for reducing microbial numbers on the surface of human body wherein the zinc carboxylate is a salt of a fatty acid having from 6 to 14 carbon atoms, **characterised in that** the reduction in microbial numbers leads to a reduction in malodour.

2. Non-therapeutic use according to claim 1 wherein the number of bacteria on the surface of the human body is decreased.

3. Non-therapeutic use according to claim 2 wherein the number of *Staphylococcus* bacteria on the surface of the human body is decreased.

4. Use according to claim 3 wherein the number of *Staphylococcus hominis* bacteria on the surface of the human body is decreased.

5. Use according to any of the preceding claims wherein the zinc carboxylate salt also entraps malodour molecules, enabling double protection from malodour.

6. Use according to any of the preceding claims wherein the zinc carboxylate is a salt of a saturated fatty acid.

7. Use according to any of the preceding claims wherein the zinc carboxylate is a salt of a branched chain fatty acid.

8. Use according to any of the preceding claims wherein the zinc carboxylate is a salt of a fatty acid having from 8 to 12 carbon atoms.

9. Use according to any of the preceding claims wherein the zinc carboxylate is a liquid at 20°C and atmospheric pressure.

10. Use according to claim 9 wherein the zinc carboxylate salt is zinc neodecanoate.

11. Use according to any of the preceding claims wherein the zinc carboxylate salt is topically applied to the underarm regions of human body.

12. A zinc carboxylate salt for therapeutic use as an antimicrobial agent on the surface of a human body, wherein the zinc carboxylate is a salt of a fatty acid having from 6 to 14 carbon atoms, **characterised in that** the reduction in microbial numbers leads to a reduction in malodour.

13. A zinc carboxylate salt for therapeutic use according to claim 12 wherein said use results in the reduction of *Staphylococcus* bacteria on the surface of the human body.

14. A zinc carboxylate salt for therapeutic use according to claim 13 wherein said use results in the reduction of *Staphylococcus hominis* bacteria on the surface of the human body.

## Patentansprüche

1. Nicht-therapeutische Verwendung eines Zinkcarboxylatsalzes zur Verringerung der Keimzahl auf der Oberfläche des menschlichen Körpers, wobei das Zinkcarboxylat ein Salz einer Fettsäure mit 6 bis 14 Kohlenstoffatomen ist, **dadurch gekennzeichnet, dass** die Verringerung der Keimzahl zu einer Verringerung von üblem Geruch führt.

2. Nichttherapeutische Verwendung nach Anspruch 1, wobei die Anzahl der Bakterien auf der Oberfläche des menschlichen Körpers verringert wird.

3. Nichttherapeutische Verwendung nach Anspruch 2, wobei die Anzahl der Staphylococcus-Bakterien auf der Oberfläche des menschlichen Körpers verringert wird.

4. Verwendung nach Anspruch 3, wobei die Anzahl der Staphylococcus hominis-Bakterien auf der Oberfläche des menschlichen Körpers verringert wird.

5. Verwendung nach einem der vorhergehenden Ansprüche, wobei das Zinkcarboxylatsalz auch Geruchsmoleküle einschließt, wodurch ein doppelter Schutz vor üblem Geruch ermöglicht wird.

6. Verwendung nach einem der vorhergehenden Ansprüche, wobei das Zinkcarboxylat ein Salz einer gesättigten Fettsäure ist.

7. Verwendung nach einem der vorhergehenden Ansprüche, wobei das Zinkcarboxylat ein Salz einer verzweigtkettigen Fettsäure ist.

8. Verwendung nach einem der vorhergehenden Ansprüche, wobei das Zinkcarboxylat ein Salz einer Fettsäure mit 8 bis 12 Kohlenstoffatomen ist.

9. Verwendung nach einem der vorhergehenden Ansprüche, wobei das Zinkcarboxylat bei 20°C und Atmosphärendruck flüssig ist.

10. Verwendung nach Anspruch 9, wobei das Zinkcarboxylatsalz Zinkneodecanoat ist.

11. Verwendung nach einem der vorhergehenden Ansprüche, wobei das Zinkcarboxylatsalz topisch auf die Achselhöhlen des menschlichen Körpers aufgetragen wird.

12. Zinkcarboxylatsalz zur therapeutischen Verwendung als antimikrobielles Mittel auf der Oberfläche des menschlichen Körpers, wobei das Zinkcarboxylat ein Salz einer Fettsäure mit 6 bis 14 Kohlenstoffatomen ist, **dadurch gekennzeichnet, dass** die Verringerung der Keimzahl zu einer Verringerung von üblem Geruch führt.

13. Zinkcarboxylatsalz zur therapeutischen Verwendung nach Anspruch 12, wobei die Verwendung zu einer Verringerung von Staphylococcus-Bakterien auf der Oberfläche des menschlichen Körpers führt.

14. Zinkcarboxylatsalz zur therapeutischen Verwendung nach Anspruch 13, wobei die Verwendung zu einer Verringerung von Staphylococcus hominis-Bakterien auf der Oberfläche des menschlichen Körpers führt.

## Revendications

1. Utilisation non thérapeutique d'un sel carboxylate de zinc pour réduire les comptes microbiens sur la surface d'un corps humain, dans laquelle le carboxylate de zinc est un sel d'un acide gras ayant de 6 à 14 atomes de carbone, **caractérisée en ce que** la réduction des comptes microbiens conduit à une réduction des mauvaises odeurs.

2. Utilisation non thérapeutique selon la revendication 1, dans laquelle le nombre de bactéries sur la surface du corps humain est réduit.

3. Utilisation non thérapeutique selon la revendication 2, dans laquelle le nombre de bactéries *Staphylococcussur* sur la surface du corps humain est réduit.

4. Utilisation selon la revendication 3, dans laquelle le nombre de bactéries *Staphylococcus hominis* sur la surface du corps humain est réduit.

5. Utilisation selon l'une quelconque des revendications précédentes, dans laquelle le sel carboxylate de zinc également piège les molécules de mauvaises odeurs, en permettant une double protection contre les mauvaises odeurs.

6. Utilisation selon l'une quelconque des revendications précédentes, dans laquelle le carboxylate de zinc est un sel d'un acide gras saturé.

7. Utilisation selon l'une quelconque des revendications précédentes, dans laquelle le carboxylate de zinc est un sel d'un acide gras à chaîne ramifiée.

8. Utilisation selon l'une quelconque des revendications précédentes, dans laquelle le carboxylate de zinc est un sel d'un acide gras ayant de 8 à 12 atomes de carbone.

9. Utilisation selon l'une quelconque des revendications précédentes, dans laquelle le carboxylate de zinc est un liquide à 20°C et sous la pression atmosphérique.

10. Utilisation selon la revendication 9, dans laquelle le sel carboxylate de zinc est le néodécanoate de zinc.

11. Utilisation selon l'une quelconque des revendications précédentes, dans laquelle le sel carboxylate de zinc est appliqué par voie topique aux régions des aisselles du corps humain.

12. Sel carboxylate de zinc pour une utilisation thérapeutique en tant qu'agent antimicrobien sur la surface d'un corps humain, dans lequel le carboxylate de zinc est un sel d'un acide gras ayant de 6 à 14 atomes de carbone, **caractérisé en ce que** la réduction des comptes microbiens conduit à une réduction des mauvaises odeurs.

13. Sel carboxylate de zinc pour une utilisation thérapeutique selon la revendication 12, dans lequel ladite utilisation a pour résultat la réduction de bactéries *Staphylococcus* sur la surface du corps humain.

14. Sel carboxylate de zinc pour une utilisation thérapeutique selon la revendication 13, dans lequel ladite utilisation a pour résultat la réduction de bactéries *Staphylococcus hominis* sur la surface du corps humain.
